# EUROPEAN PATENT APPLICATION

(11) **EP 2 952 179 A1**
(43) Date of publication of application: **09.12.2015**
(21) Application number: 14171405.5
(22) Date of filing: 05.06.2014
(51) Int. Cl.: A61K 9/12, A61K 31/19, A61Q 19/00, B65D 83/28, A61B 18/02, A61M 35/00

(54) **Treatment of a skin lesion**

(71) Applicant: Medical Brands Research B.V., 1019 HC Amsterdam (NL)
(72) Inventor: Hendriks, Maikel, 1019 HC Amsterdam (NL)
(74) Representative: Ellens, Andries

(57) **Abstract**

The invention provides a spray applicator (1) for topical treatment, the spray applicator (1) comprising a container (2) comprising a treatment liquid (52) under pressure and a propellant (51), wherein the container (2) is in fluid connection with a spray valve system (4) for application of at least part of the treatment liquid (52) on a topical area, wherein the treatment liquid (52) comprises an acid, wherein the container (2) comprises a container wall (20) having a top layer (22) and a top layer coating (21) on said top layer (22), wherein the top layer (22) comprises a metal and wherein the top layer coating (21) comprises a polymer.

## Description

### FIELD OF THE INVENTION

The invention relates to a spray applicator, the use of such spray applicator and the use of the treatment liquid (that is contained in the spray applicator). The invention further relates to a composition comprising said treatment liquid for cosmetical and/or medical use. Further the invention relates to a method for providing said spray applicator.

### BACKGROUND OF THE INVENTION

Various compositions are known for the treatment of skin lesions, such as warts, corn and calluses, actinic keratosis, keratosis pilaris, acne, skin hyperpigmentation and/or nail lesions, such as ingrown toenails and/or lesions in mucous membranes, such as cold sores and mouth ulcers. WO2012007584, for instance, describes a composition for the treatment of superficial lesions, in particular skin lesions, mucous membrane lesions and/or nail lesions, an applicator comprising such a composition and the use of such a composition. The composition comprises an effective amount of trichloroacetic acid, at least one thickener, and at a physiologically acceptable solvent. The composition is considered effective against a plethora of superficial lesions selected from the group consisting of viral warts, verrucae, water warts (molluscum contagiosum), corns and calluses, and skin hyperpigmentation: age spots, solar lentigo, senial lentigo, acne, keratosis pilaris, actinic keratosis, mouth ulcers (canker sores), cold sores, ingrown toenails, onychomycosis, eyelid xanthelasma.

### SUMMARY OF THE INVENTION

Many prior art method to treat warts or other skin lesions are not effective or not effective enough. Further, some of the prior art methods need specific care and medical supervision, such as the treatment of warts with liquid nitrogen.

Hence, it is an aspect of the invention to provide an alternative method and/or applicator, which preferably further at least partly obviate one or more of above-described drawbacks, and which is especially safe.

In a first aspect, the invention provides a spray applicator for topical treatment, the spray applicator comprising a container comprising a treatment liquid under pressure and a propellant, wherein the container is in fluid connection with a spray valve system, especially a dosage controlled spray valve system, for application of at least part of the treatment liquid on a body surface (herein also indicated as topical area), wherein the treatment liquid comprises an acid, wherein the container comprises a container wall having a top layer and optionally a top layer coating on said top layer. In a specific embodiment, the top layer comprises a metal and the top layer coating comprises in an embodiment a polymer, such as polypropylene or polyethylene. In yet a further aspect, the invention provides a spray applicator, especially for topical treatment, the spray applicator comprising a container comprising a treatment liquid under pressure and a propellant, wherein the container is in fluid connection with a spray valve system (also comprised by the spray applicator) for application of at least part of the treatment liquid on a topical area, wherein the treatment liquid comprises an acid, wherein the container comprises a container wall may comprise aluminum and a coating comprising a polymer, wherein the spray applicator further especially comprises a distance holder configured to arrange on a topical area and comprising a opening configured to enclose a topical lesion, wherein the spray applicator is configured to spray the treatment liquid through the opening in the distance holder, especially thereby reducing exposure of topical areas that need not be exposed to the treatment liquid. At least part of such (healthy) topical area is covered by part of the distance holder (the part that surrounds the opening in the distance holder).

The invention also provides such composition per se, i.e. a composition comprising such treatment liquid and the propellant according as defined herein, especially for use in freezing and acid treatment of a topological lesion, such as in a cosmetic treatment. The invention also provides such composition comprising such treatment liquid and the propellant according as defined herein, especially for use in freezing and acid treatment of a topological lesion, such as in a medical treatment. The invention further provides such composition especially for use in the treatment of a wart (or corn). Further, the invention also provides such treatment liquid per se, especially for use in freezing and acid treatment of a topological lesion, such as in a cosmetic treatment. The invention also provides such treatment liquid, especially for use in freezing and acid treatment of a topological lesion, such as in a medical treatment. The invention further provides such treatment liquid especially for use in the treatment of a wart (or corn).

The acid in the treatment liquid is especially applied to peel the skin or mucous membrane, especially the skin, by which the skin or mucous membrane may renew itself. The propellant is not only used to provide the spray, but may especially advantageously also cool the treatment liquid and/or the topical lesion. The treatment liquid and the propellant may both have an advantageous effect on the lesion to be treated, such as a wart (or corn): freezing, due to the propellant, may induce blister formation and the acid may have a peeling effect. This may thus lead to a freezing effect, in addition to the acid treatment or acid effect. It appears that this combined treatment is more efficient than the separate treatments alone. Further, the invention may allow a more effective treatment than conventional cryotherapy but may be more efficient than traditional wart acid treatments. The treatment liquid, especially the acid, may provide a oxidation effect to the lesion.

The spray applicator or the treatment liquid, or the treatment liquid in combination with the propellant, may be used for several types of lesions. The spray applicator, or the treatment liquid, or the treatment liquid in combination with the propellant, may be applied for medical use and/or non-medical use, such as cosmetical use. Herein, the term "treatment liquid" refers to the liquid comprising the acid and optionally one or more other components, and the term "treatment composition" refers to the composition or mixture comprising the treatment liquid and the propellant, which is applied on the topical area (and which escapes as spray yet from the spray valve system).

In a further aspect, the invention especially provides the use of the spray applicator, as defined herein, for a (cosmetical) treatment of a topical lesion selected from the group consisting of epidermodysplasia veruciformis, a HPV (human papillomavirus) caused skin disorder, acne, a scar, a wrinkle, a tattoo, eyelid xanthelasma, an age spot, a senial lentigo, a solar lentigo, a melasma, a hand wart, a plantar wart, a corn, an ingrown toe nail, a mucous membrane lesion. The term "wart" may e.g. relate to one or more of verruca vulgaris and verruca plantaris. The treatment liquid is especially suitable for the treatment of one or more of a wart and a corn. Likewise, also the treatment liquid per se may be used. Hence, the invention provides in yet a further aspect also the use of the treatment liquid as defined herein, for a cosmetical treatment (or a medical treatment) of a topical lesion selected from the group consisting of epidermodysplasia veruciformis, a HPV caused skin disorder, acne, a scar, a wrinkle, a tattoo, eyelid xanthelasma, an age spot, a senial lentigo, a solar lentigo, a melasma, a hand wart, a plantar wart, a corn, an ingrown toe nail, a mucous membrane lesion.

The term topical treatment especially relates to the treatment with the treatment liquid (and propellant) to a body surface, such as the skin or a mucous membrane, especially the (human) skin.

In a specific embodiment, the spray applicator one or more of the following conditions, especially all, may apply: (i) the acid comprises one or more acids selected from the group consisting of trichloro acetic acid, salicylic acid, formic acid, glycolic acid, dichloro acetic acid, monochloro acetic acid, boric acid, nitric acid, sulfuric acid, phosphoric acid, oxalic acid, lactic acid, hydrochloric acid; (ii) the propellant comprises one or more propellants selected from the group consisting of dimethyl ether (DME), diethyl ether (DEE), methyl ethyl ether (methoxy ethane), carbon dioxide (CO₂), nitrogen (N₂), nitrous oxide, propane, butane, liquified petroleum gases (LPG), and a chlorofluorcarbon (CFC); (iii) the top layer coating comprises a polyolefin, especially one or more of polyethylene and polypropylene; and (iv) wherein the metal of the top layer comprises aluminum. Even more especially, the treatment liquid comprises at least trichloroacetic acid (TCA) and wherein the propellant comprises at least one or more of dimethyl ether (DME), diethyl ether (DEE), and methyl ethyl ether (methoxy ethane). Hence, the acid may also comprise a combination of two or more different acids. Further, also the propellant may comprise a combination of two or more propellants.

The top layer coating, when available, or the top layer, in case no coating is available, especially comprises an acid resistant material. Especially good materials for the top layer coating may be selected from the group consisting of chlorinated polyvinyl chloride (CPVC), ethylene chlorotrifluoroethylene (ECTFE), (enhanced) polytetrafluoroethylene, (enhanced PTFE), high-density poly ethylene (HDPE), polyether ether ketone (PEEK), polypropylene (PP), polysulfone (PSU), polyphenylene sulfide (PPS), polyvinyl chloride (especially type 1 (PVC, Type 1) or type 2 (PVC, Type 2)), polyvinylidene fluoride (PVDF), polytetrafluoroethylene (PTFE), Polyamide-imide (PAI), ultra high molecular weight polyethylene (UHMW). These materials are especially resistant against the acid content of the container. These materials may however also be used as wall of the container (and thus the top layer comprising such material).

Especially, the propellant has a boiling point of less than 10 °C, or even less than -20 °C. Further, in a specific embodiment the pressure in the container is in the range of 1.5-10 bar, like 4-10 bar, such as especially at least 5 bar.

Alternatively, the propellant, or optionally a further component of the treatment liquid, may have a heating effect (on the topical area (such as the skin) to which the treatment composition is applied. Both a propellant having a cooling effect and a propellant having a heating effect (or a further component having such effect), may lead to blister formation. Hence, optionally an exothermic effect on the topical area may be achieved. This (also) facilitates e.g. the removal process of the wart (or corn). For instance, after the application of the treatment liquid, after some time the wart may fall off.

Of course, the treatment liquid may optionally comprise one or more other ingredients, including one or more other active ingredients. The composition as described herein may comprise other ingredients commonly used in cosmetics and pharmaceutical products, such as one or more of a surfactant, a colorant and a perfume.

Trichloroacetic acid (TCA) in the treatment liquid as described herein, proved to be effective against a plethora of skin lesions, in particular warts, corn and calluses, molluscum contagiosum, acne, skin hyperpigmentation, actinic keratosis as well as nail lesions including ingrown toenails and onchyomycosis, and lesions in mucous membranes such as mouth ulcers and cold sores. Application of trichloroacetic acid is particularly effective against genital warts, and especially against ano-genital warts where it has been studied in comparative trials where its effectiveness was compared with other anti-wart therapies. In addition, TCA application was effective various other skin disorders, in particular: epidermodysplasia veruciformis- skin disorder also caused by HPV virus, acne, to remove acne scars and wrinkles, tattoo removal, eyelid xanthelasma, age spots, senial lentigo and solar lentigo, melasma. Additionally or alternatively, the treatment liquid may comprise one or more of formic acid and salicylic acid, which alone or in combination may also provide a good result. Hence, in an embodiment the treatment liquid comprises TCA, and optionally formic acid, and in another embodiment the treatment liquid comprises formic acid, and optionally TCA.

Salicylic acid has a positive effect in treating skin and nails. In addition to that, salicylic acid gives a mild anaesthetic effect. Salicylic acid in combination with TCA showed a synergistic effect against skin lesions and nail lesions. Another advantage is that using a combination of salicylic acid and TCA allows for a composition with a relatively low concentration of each of salicylic acid and TCA with a similar effect to compositions using only TCA or only salicylic acid. Using the relatively low concentrations of TCA and salicylic acid decreases the chance of skin irritation due to either of these compounds. Moreover, salicylic acid diminished the discomforting burning feeling on skin and nails sometimes experienced by persons treated with products containing substantial amounts of TCA.

The treatment liquid may thus amongst others be used in for instance a skin peeling treatment, for the medical or cosmetic treatment of skin lesion selected from the group consisting of warts, corn and calluses, and for nail treatments including ingrown toenails. A postulated mechanism of action is that the composition comprising TCA, or another acid, softens the skin or nail, and enables to peel the skin or nail lesion away. For severe lesions, multiple treatments may be needed. Both cosmetic and medical treatments may be performed with the compositions according to the invention. Other active ingredients contributing to the treatment of the lesion may be added. For instance salicylic acid (see also above) is another component effective in corroding the skin or nails, and may be used as an additional active ingredient in combination with trichloroacetic acid. The freezing effect of the propellant seems to enhance these mechanisms, or at least the peeling mechanism, as it facilitates blister formation. Hence, the invention further provides the use of a treatment liquid for the cosmetic treatment of the human skin. Especially, the composition is used for a skin peeling treatment or the treatment of a skin lesion selected from the group consisting of warts, corn and calluses. The compositions are particularly effective against warts and related lesions, including viral warts, verrucae, and water warts (molluscum contagiosum). These treatments are typically considered cosmetic treatments rather than medical treatments. The invention also provides the use of a composition as described herein for the cosmetic treatment of nails. In particular nail deformations (for instance as a result from onchyomycosis) and ingrown toenails may be treated effectively.

The invention further provides the use of a treatment liquid or treatment composition as described herein for the treatment of lesions in mucous membranes, in particular mouth ulcers and cold sores. The invention further provides the use of the treatment liquid or treatment composition as described herein for the cosmetic treatment of superficial lesions selected from the group consisting of viral warts, verrucae, water warts (molluscum contagiosum), corns and calluses, and age spots, solar lentigo, senial lentigo, acne, mouth ulcers (canker sores), cold sores ingrown toenails

The treatment liquid especially comprises an aqueous liquid, such as especially water, and the acid. Especially, the total amount of other components in the treatment liquid, other than the acid and the liquid, such as the aqueous liquid, is less than 10 wt.%, especially less than 5 wt.%, examples of other compounds, such as a colorant or a perfume, are mentioned above. Especially, the treatment liquid comprises the acid in a concentration up to 40 wt.%, such as at maximum 35 wt.%, even more especially at maximum 20 wt.%. Higher acid concentrations may lead to undesired topological effects and/or may be unsafe, such as in the kind of application with children or specific topical parts, like mucous membrane. In a specific embodiment, the treatment liquid comprises the acid in a concentration in the range of 1-10 wt.%. Lower concentrations than 1 wt.% may not be effective enough. Especially, the concentration of the acid in the treatment liquid is at least 2 wt.%, even more especially at least 3 wt.%.

The spray applicator allows without contact of the spray valve system of the applicator with the skin, etc., the treatment of the topological lesion. This is safer and also more hygienic than applicators that need contact with the lesion. The spray applicator may especially be configured to provide a single spray dose of in embodiments not more than 1 ml, such as especially not more than 0.75 ml, such as 0.1-0.5 ml, like 0.1-0.2 ml, or less than 0.2 ml. As will be known by the person skilled in the art, this can be tuned by amongst others the nozzle (outlet) dimensions and the pressure of the propellant in the container. A dosage valve or dosage application may be used to control that the desired spray dose is substantially always met. In some instances, especially in view of safety, a narrow spray may be desirable. Hence, in an embodiment the spray applicator is configured to provide a spray having a spray angle (opening angle) (θ) wherein 90% of a spray dose escapes from an outlet of the spray valve system, wherein the spray angle (θ) is in the range of 15-45°. The spray angle may be tuned by the dimensions of the nozzle. For instance, the nozzle (of the spray valve system) may comprise a conical channel.

In view of safety, especially child safety, the spray valve system may in an embodiment include an infant opening protection. For instance, infant opening protection system may include a turning system that has a state wherein the spray valve system is blocked and a state wherein the spray valve system can be applied.

The container comprises a container wall. The container comprises a top layer or top surface, i.e. the outer part of the wall, which is in physical contact with the treatment liquid and/or propellant when the container is filled. This wall may be entirely of a metal such as aluminum. In such instance, the top layer is by definition aluminum. Hence, the container wall may be made of aluminum. On top of this top layer, especially in view of life time of the spray applicator, the container wall comprises a coating. It appears that a polymer coating, especially selected from the group consisting of polypropylene and polyethylene provide good properties. The coating may include a single-layer coating or a multi-layer coating. In case of the latter coating, adjacent layers of the multi-layer coating may differ in composition and/or thickness. The polypropylene polymer may e.g. be selected from the type of homopolymer, random copolymer, and block copolymer. The comonomer is typically used with ethylene. Likewise, the polyethylene polymer may e.g. be selected from the type of homopolymer, random copolymer, and block copolymer. Examples of PE may e.g. be selected from the group consisting of ultra-high-molecular-weight polyethylene (UHMWPE), high-density polyethylene (HDPE), cross-linked polyethylene (PEX or XLPE), medium-density polyethylene (MDPE), linear low-density polyethylene (LLDPE), low-density polyethylene (LDPE), and very-low-density polyethylene (VLDPE). Hence, the container wall may comprise a metal (layer), especially aluminum, and an inner surface coating comprising a polymer coating. However, alternatively, the container wall may comprise a polymer, such as one of the above indicated polymers, or one or more of the above indicated acid resistant polymers. In a further embodiment, the container wall comprises a polymer and a polymer top coating, such as one or more of the above indicated acid resistant polymers.

In a further embodiment, the spray applicator may comprise a distance holder configured to arrange on a topical area, such as the skin, wherein the distance holder comprises an opening configured to enclose a topical lesion. The user may thus arrange the spray applicator on the skin with the opening surrounding the skin lesion. In this way, exposure of healthy skin to the treatment liquid may be minimized, but hereby it may also be prevented that the spray valve system comes into contact with the topical lesion (such contact is not desirable). The distance holder is an element that can be used to arrange the spray applicator to a topical area with a (more) controlled distance from the spray valve system to the topical lesion to be sprayed with the treatment liquid. In this way, the dosage the lesion receives can better controlled than with a manual arrangement of the spray applicator over the lesion, in addition the distance will also contribute to the effectiveness since the mixture has time to react with the oxygen in which the transfer of the cold will be promoted. Hence, especially the spray applicator is configured to spray the treatment liquid through the opening in the distance holder. Further, the distance holder is configured to prevent physical contact between the spray valve system and the topical area. Optionally, the opening of the distance holder is adjustable in size. For instance, the distance holder may include an adjustable diaphragm (as opening). In this way, even more the healthy topical area may be protected and efficient and targeted application of treatment liquid to the desired skin lesion is ensured. The distance holder may be integrated in the spray applicator, or may be an attachable unit, e.g. with Snap-On snap-off elements. The distance holder may include a transparent part, that facilitates visual inspection by a user of the area that is sprayed. Hence, the applicator may be designed to ensure a visual control during the use of the applicator to ensure a safe and effective treatment. Alternatively or additionally, the distance holder may be configured in such a way that a user may visual inspect the area that is sprayed when used by the user himself or herself. The opening in the distance holder may be round or square or oval, or have another shape. Especially, part of the distance holder is configured to fit on a human body. Optionally, a plurality of distance holders may be provided, which may fit to different parts of the body.

In yet a further aspect, the invention also provides a method of providing the spray applicator, especially as defined herein, the method comprising:
- premixing acid and an aqueous liquid and providing the mixture to the container;
- providing the propellant in a liquid phase to the container; and
- closing the container with the spray valve system (and arrange a manual applicator to the spray valve system).

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to a device comprising one or more of the characterizing features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### BRIEF DESCRIPTION OF THE DRAWINGS

Embodiments of the invention will now be described, by way of example only, with reference to the accompanying schematic drawings in which corresponding reference symbols indicate corresponding parts, and in which:

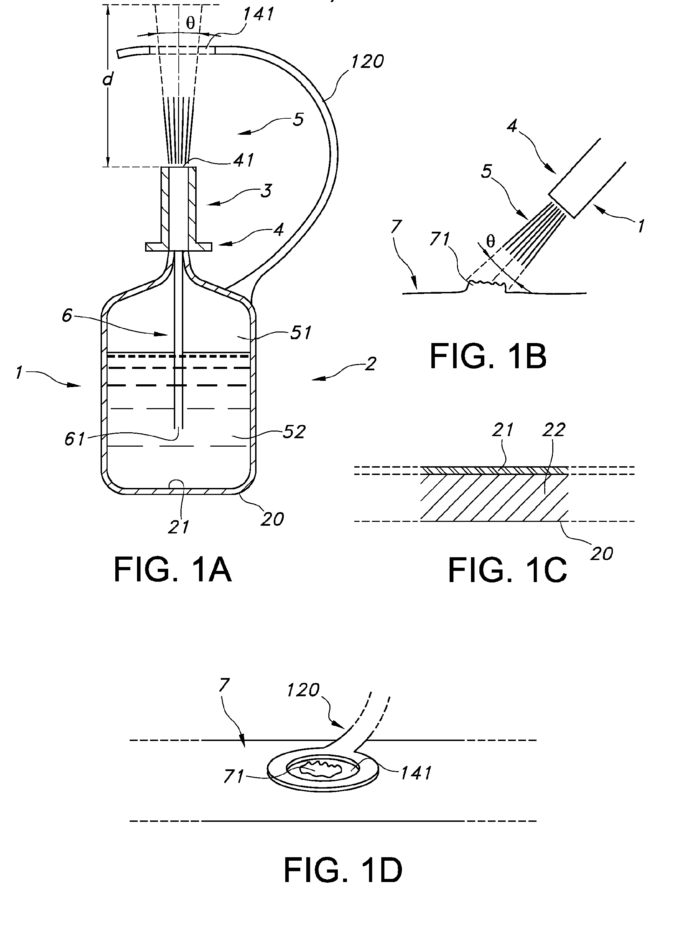

Figs.1a-1d schematically depicts embodiments and aspects of the spray applicator.

The drawings are not necessarily on scale.

### DETAILED DESCRIPTION OF THE EMBODIMENTS

Fig. 1a schematically depicts a embodiment of the spray applicator 1. The spray applicator is especially an aerosol spray applicator. The spray applicator 1 comprises a can or container 2 as well as a spray valve system 4 with a nozzle or outlet 41. The container 2 comprises a container wall 20, which may optionally include a container wall coating 21. Further, optionally integrated with the valve system 4, the spray system 4 comprises a manual applicator. For instance by pressing this manual applicator, the spray valve systems frees treatment liquid 52 from the container 2 which is under elevated pressure due to the presence of a propellant under pressure. The spray valve system 4 has an opening or nozzle 41. The propellant is indicated with reference 51. Part of the propellant may also be in the liquid phase. The spray applicator may further comprise a dip tube 6, in fluid connection with the spray valve system 4 and with a dip tube opening 61, which during most of the lifetime of the spray applicator 1 will be emerged in the treatment liquid.

In an embodiment, the spray applicator 1 may especially be configured to provide a single spray dose of not more than 0.75 ml. Further, the spray applicator 1 may be configured to provide a spray 5 having a spray angle θ wherein 90% of a spray dose escapes from an outlet 41 of the spray valve system 4, wherein the spray angle θ is in the range of 15-45°. These spray characteristics may especially be obtained within a distance d of the nozzle 41, such as within a distance of 0-5 cm, like 0.5-5 cm, such as 0.5-2 cm. The spray valve system may include a metering valve. Especially, the spray valve system 4 is configured to administer the treatment liquid in a metered manner.

Optionally, the spray applicator 1 may include a distance holder 120, which may further include an opening 141. With such applicator, the opening may be arranged around the wart, and the jet may only provided to the topical area of the opening (see also Fig. 1d). Optionally, the distance holder includes transparent regions, such as openings or transparent material, thereby facilitating the user to easier perceive the topical area that is (to be) treated.

Fig. 1a is a very schematic embodiment. Of course, the spray applicator may include a container in a holder. Such holder may for instance host the optional distance holder 120. The distance holder 120 may be integrated in the spray applicator 1 or may be temporarily connected to the spray applicator. Optionally the distance holder 120 is bendable and can be arranged in a position for use and a position for rest. The former position may e.g. provide a smaller spray applicator (for transport purposes).

Fig. 1b schematically depicts how the spray applicator 1 can be used. For the sake of simplicity, only the valve system 4 with nozzle 41 is shown. The spray or spray het 5 may be applied to a lesion 71, such as a wart, on a body surface or topical area 7, such as the skin.

Fig. 1c schematically depicts part of the container wall of the container. The container wall 20 may optionally comprise a container wall coating 21. This coating will thus be in contact with the treatment liquid. A top layer of the container wall 20 is indicated with reference 22. The container wall may e.g. be of aluminum and thus the top layer 22 is of aluminum. On the top layer 22, optionally the coating 21 may be available, for e.g. a PP coating.

Fig. 1d schematically depicts how the distance holder 120 can be arranged to a topical region or body surface 7, such as the skin, with the skin lesion 71, such as a wart surrounded by the opening 141. This allows dosing the treatment liquid to the opening only, thus the topical region in the opening only, and substantially not to healthy topical regions. Optionally, the opening is scalable, i.e. the opening area may be adapted by the user. For instance a diaphragm type of opening may be used.

Hence, the invention provides amongst others a composition for treatment of skin lesions by using a special designed (1) coated aluminum container, (2) a dosage delivery valve and nozzle, (3) a targeted applicator, (4) an active medical serum (acid + freezing agent), and optionally (5) a child safety lock. For instance, a composition for treatment of superficial lesions may be provided, wherein a freezing agent is applied having a low boiling point and an (extreme) skin exfoliating acid, such as acetic acid. Further, the invention provides a device for the application of the active serum on the skin lesion, wherein the device comprises an applicator to include a dosage delivery nozzle to apply, in an acid compliant coated aluminum container consisting of a (tubular) container and (cylindrical) body with a targeted open applicator which ensures that the dosage valve is released at a certain distance from the skin to allow oxidization for controlled temperature of the freezing agent, along with the active serum to treat the skin lesion. TCA may advantageously be provided as powder, which can be combined with a liquid to provide the treatment liquid.

## Claims

1. A spray applicator (1) for topical treatment, the spray applicator (1) comprising a container (2) comprising a treatment liquid (52) under pressure and a propellant (51), wherein the container (2) is in fluid connection with a spray valve system (4) for application of at least part of the treatment liquid (52) on a topical area, wherein the treatment liquid (52) comprises an acid, wherein the container (2) comprises a container wall (20) comprising aluminum and a coating (21) comprising a polymer, wherein the spray applicator (1) further comprises a distance holder (120) configured to arrange on a topical area and comprising a opening (141) configured to enclose a topical lesion, wherein the spray applicator is configured to spray the treatment liquid (52) through the opening (141) in the distance holder (120).

2. The spray applicator (1) according to claim 1, wherein the treatment liquid (52) at least comprises trichloro acetic acid.

3. The spray applicator (1) according to claim 1, wherein
- the acid comprises one or more acids selected from the group consisting of trichloro acetic acid, salicylic acid, formic acid, glycolic acid, dichloro acetic acid, monochloro acetic acid, boric acid, nitric acid, sulfuric acid, phosphoric acid, oxalic acid, lactic acid, hydrochloric acid,
- the propellant comprises one or more propellants selected from the group consisting of dimethyl ether (DME), diethyl ether (DEE), methyl ethyl ether (methoxy ethane), carbon dioxide (CO₂), nitrogen (N₂), nitrous oxide, propane, butane, liquified petroleum gases (LPG), and a chlorofluorcarbon (CFC),
- the top layer coating (21) comprises one or more of polyethylene and polypropylene; and
- wherein the metal of the top layer (22) comprises aluminum.

4. The spray applicator (1) according to any one of the preceding claims, wherein the treatment liquid comprises at least trichloroacetic acid (TCA) and wherein the propellant comprises at least one or more of dimethyl ether (DME), diethyl ether (DEE), and methyl ethyl ether (methoxy ethane).

5. The spray applicator (1) according to any one of the preceding claims, wherein the spray valve system (4) includes an infant opening protection.

6. The spray applicator (1) according to any one of the preceding claims, wherein the propellant has a boiling point of less than 10 °C, and wherein pressure in the container (2) is in the range of 1.5-10 bar.

7. The spray applicator (1) according to any one of the preceding claims, wherein the treatment liquid (52) comprises the acid in a concentration up to 40 wt.%, especially wherein the treatment liquid (52) comprises the acid in a concentration in the range of 1-10 wt.%.

8. The spray applicator (1) according to any one of the preceding claims, wherein the spray applicator (1) is configured to provide a single spray dose of not more than 0.75 ml.

9. The spray applicator (1) according to any one of the preceding claims, wherein the distance holder is configured to prevent physical contact between the spray valve system (4) and the topical area, wherein the opening (141) is adjustable in size.

10. The spray applicator (1) according to any one of the preceding claims, wherein the spray applicator (1) is configured to provide a spray (5) having a spray angle (θ) wherein 90% of a spray dose escapes from an outlet (41) of the spray valve system (4), wherein the spray angle (θ) is in the range of 15-45°.

11. Use of the spray applicator (1) according to any one of the preceding claims for a cosmetical treatment of a topical lesion selected from the group consisting of epidermodysplasia veruciformis, a HPV caused skin disorder, acne, a scar, a wrinkle, a tattoo, eyelid xanthelasma, an age spot, a senial lentigo, a solar lentigo, a melasma, a hand wart, a plantar wart, a corn, an ingrown toe nail, a mucous membrane lesion.

12. Use of the treatment liquid (52) as defined in any one of claims 1-10, for a cosmetical treatment of a topical lesion selected from the group consisting of epidermodysplasia veruciformis, a HPV caused skin disorder, acne, a scar, a wrinkle, a tattoo, eyelid xanthelasma, an age spot, a senial lentigo, a solar lentigo, a melasma, a hand wart, a plantar wart, a corn, an ingrown toe nail, a mucous membrane lesion.

13. A composition comprising the treatment liquid (52) and the propellant as defined in any one of claims 1-10, for use in freezing and acid treatment of a topological lesion.

14. The composition according to claim 13, for use in the treatment of a wart or a corn.

15. A method of providing the spray applicator (1) according to any of the claims 1-10, the method comprising:
- premixing acid and an aqueous liquid and providing the mixture to the container (2);
- providing the propellant in a liquid phase to the container (2); and
- closing the container with the spray valve system (4).
